# EUROPEAN PATENT SPECIFICATION

(11) **EP 2 776 111 B1**
(45) Date of publication and mention of the grant of the patent: **30.12.2020**
(21) Application number: 12790691.5
(22) Date of filing: 26.10.2012
(51) Int. Cl.: A61M 25/09, A61B 5/0215

(54) **SENSOR GUIDE WIRE**
SENSORFÜHRUNGSDRAHT
FIL-GUIDE POUR CAPTEUR

(30) Priority: 09.11.2011 SE 1151051; 09.11.2011 US 201161557706 P
(43) Date of publication of application: 17.09.2014
(73) Proprietor: Koninklijke Philips N.V., 5656 AG Eindhoven (NL)
(72) Inventor: DEKKER, Ronald, NL-5553 BC Valkenswaard (NL); HENNEKEN, Vincent Adrianus, NL-3544 JA Utrecht (NL); TIENSUU, Stefan, S-756 52 Uppsala (SE)
(74) Representative: Philips Intellectual Property & Standards
(86) International application number: PCT/SE2012/051165
(87) International publication number: WO 2013/070146

(56) References cited:
- EP-A1- 0 495 299
- US-A- 5 873 835
- US-A1- 2007 032 744
- US-A1- 2009 088 650

## Description

### Field of the invention

The present invention relates to a sensor guide wire for intravascular measurements of physiological variables in a living body, according to the preamble of the independent claim, and in particular to a sensor guide wire for intravascular pressure measurements.

### Background of the invention

In many medical procedures, various physiological conditions present within a body cavity need to be monitored. These physiological conditions are typically physical in nature - such as pressure, temperature, rate-of-fluid flow, and provide the physician or medical technician with critical information as to the status of a patient's condition.

One device that is widely used to monitor conditions is the blood pressure transducer. A blood pressure transducer senses the magnitude of a patient's blood pressure, and converts it into a representative electrical signal that is transmitted to the exterior of the patient. For most applications it is also required that the sensor is electrically energized.

Some means of signal and energy transmission is thus required, and most commonly extremely thin electrical cables, sometimes called microcables, are provided inside a guide wire, which itself is provided in the form of a tube, which often has an outer diameter in the order of 0.35 mm, and oftentimes is made of steel.

In order to increase the bending strength of the tubular guide wire, a core wire is positioned inside the tube. The core wire also helps to improve "pushability" and "torquability" of the guide wire. The mentioned electrical cables are e.g. positioned in the space between the inner lumen wall and the core wire.

Sensor and guide wire assemblies in which a sensor is mounted at the distal end of a guide wire are known. In U.S. Patent Re. 35,648, which is assigned to one of the present assignees, an example of such a sensor and guide wire assembly is disclosed, where a sensor and guide wire assembly comprises a sensor element, an electronic unit, a signal transmitting cable connecting the sensor element to the electronic unit, a flexible tube having the cable and the sensor element disposed therein, a solid metal wire, and a coil attached to the distal end of the solid wire. The sensor element comprises a pressure sensitive device, typically a membrane, with piezoresistive elements connected in a Wheatstone bridge-type of arrangement mounted thereon.

As is disclosed in, for example, U.S. Patent No. 6,167,763, which also is assigned to one of the present assignees, the sensor element can be arranged inside a short tube (usually referred to as a sleeve or jacket), which protects the sensor element and comprises an aperture through which the pressure sensitive device is in contact with the ambient medium. The solid metal wire - which, as also mentioned above, in the art usually is referred to as the core wire - extends through the interior of the jacket and may be provided with an enlarged diameter portion adapted for mounting of the sensor element.

A potential problem with this kind of guide wire mounted sensors is the occurrence of so-called bending artefacts. A bending artefact is a change in the output signal from the sensor that is induced by a bending of the guide wire, rather than being induced by a change in the physical environment surrounding the sensor.

To achieve the desired resistance against bending artefacts, the sensor may be designed and mounted in different ways, the common feature being that it is a cantilevered mounting arrangement that provides the desired resistance against bending artefacts.

The cantilevered mounting of the sensor, however, requires extra machining or wire forming of the core wire at the site where the sensor chip is placed. The careful machining of the core wire into different diameters and different shapes at different portions of the guide wire is a time-consuming process and may be a source of manufacturing mistakes, leading to loss of time and material in the manufacturing process.

In U.S. Patent No. 6,221,023 is disclosed a sensor mounted on the distal end of an intracorporal catheter. The sensor includes a chip that is deformable in accordance with pressure applied thereto, strain gauges mounted on the chip, a sensing plate, a projection, a cap and a tube. The projection and the cap transmit pressure applied to the catheter's distal end to the sensing plate and tilt the sensing plate in accordance with the pressure where the strain gauges issue detection signals according to the degree and the direction of the tilting.

Further sensor guide wires for intravascular measurements according to the state of the art are for instance described in US5873835A and US2009/0088650A1.

The inventors have realized that there is a need for a sensor guide wire wherein the mounting of the sensor does not involve extra machining or wire forming, which thereby is easier and less expensive to manufacture. In addition, a further need is to improve the resistance to bending artefacts, and also to improve the flexibility and bending profile of the distal portion of the sensor guide wire.

### Summary of the invention

The above-mentioned object is achieved by the present invention as defined in the independent claim.

The present invention is based upon the insight by the inventors who have realized that the stiff section of the core wire of a conventional pressure wire having the sensor element mounted in a cantilever fashion is approximately 2 mm which reduces the flexibility of the pressure sensor wire. By reducing the longitudinal extension of the stiff section of the core wire the flexibility performance is improved.

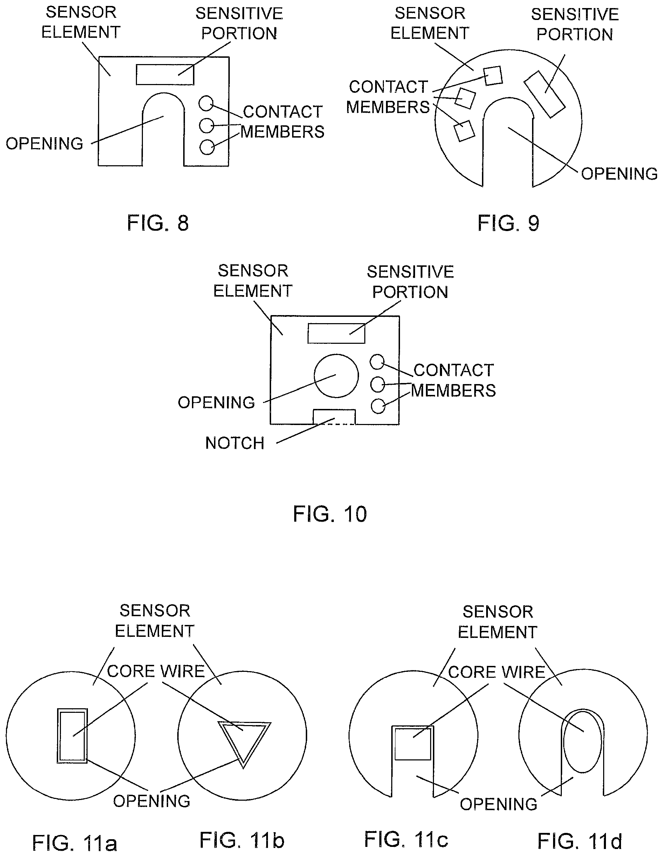

By the present invention an improved sensor guide wire is achieved which has a stiff section of the core wire in relation to the sensor element which is considerably shorter in comparison to the presently used sensor guide wire which results in a more flexible distal part of the sensor guide wire. In addition, the longitudinal extension of the jacket, arranged to protect the sensor element, may be considerably reduced which further improves the flexibility of the distal part of the sensor guide wire. As one example the longitudinal extension of the jacket may be reduced from 2.25 mm to 1.1 mm. The ability to insert the sensor guide wire into the tortuous cardiac veins is thereby improved. This is further illustrated by figure 11.

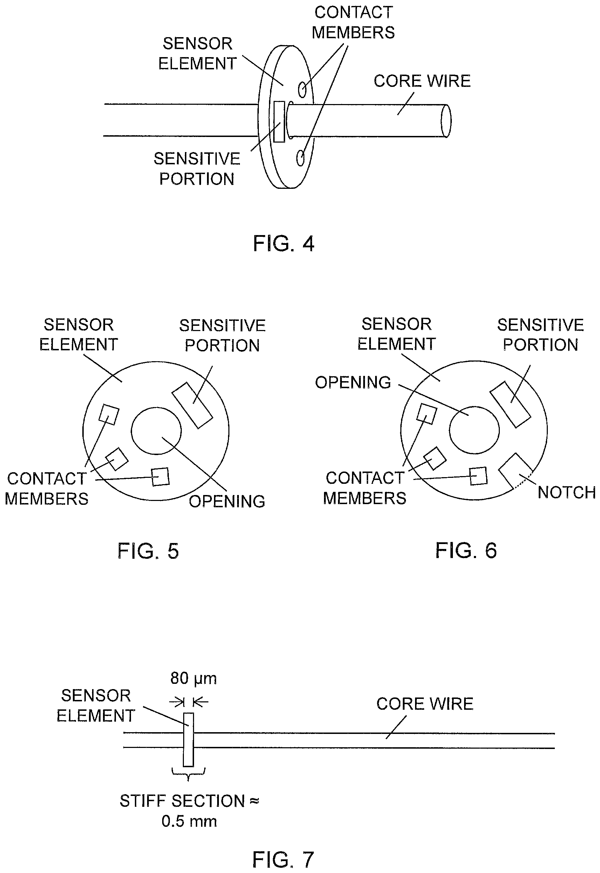

According to one embodiment the stiff section is approximately in the range of 0.3-0.5 mm. Generally the stiff section is related to the thickness of the sensor element (and also the longitudinal extension of the jacket) which preferably is in the range of 60-200 µm, preferably 80 µm. This is illustrated by figure 7 showing a schematic side view of the core wire having the sensor element mounted in accordance with the present invention.

### Short description of the appended drawings

Figure 1 illustrates the distal end of a sensor guide wire according to the prior art.
Figure 2 is a schematic side view of a core wire and a sensor element according to prior art.
Figure 3 is a schematic side view of a sensor guide wire according to the present invention.
Figure 4 is a perspective view illustrating a first embodiment of the present invention.
Figure 5 is a top view of a sensor element according to a first embodiment of the present invention.
Figure 6 is a top view of a sensor element illustrating a modified first embodiment of the present invention.
Figure 7 is side view illustrating the present invention.
Figure 8 is a top view illustrating a second embodiment of the present invention.
Figure 9 is a top view illustrating a third embodiment of the present invention.
Figure 10 is a top view illustrating a fourth embodiment of the present invention.
Figures 11a-11d are top views of a circular sensor element schematically illustrating different cross-sectional shapes of the core wire and opening.
Figures 12a-12d are top views of a rectangular sensor element schematically illustrating different cross-sectional shapes of the core wire and opening.
Figure 13 is a cross-sectional view schematically illustrating a pressure sensor according to prior art.
Figure 14 illustrates the improved flexibility of the distal part of the sensor guide wire according to the present invention.
Figure 15 shows a side view of the distal portion of a sensor guide wire according to the present invention.

### Detailed description of preferred embodiments of the invention

Throughout the application the word distal means distal in respect to the operator of the sensor guide wire and proximal means proximal in respect to the operator.

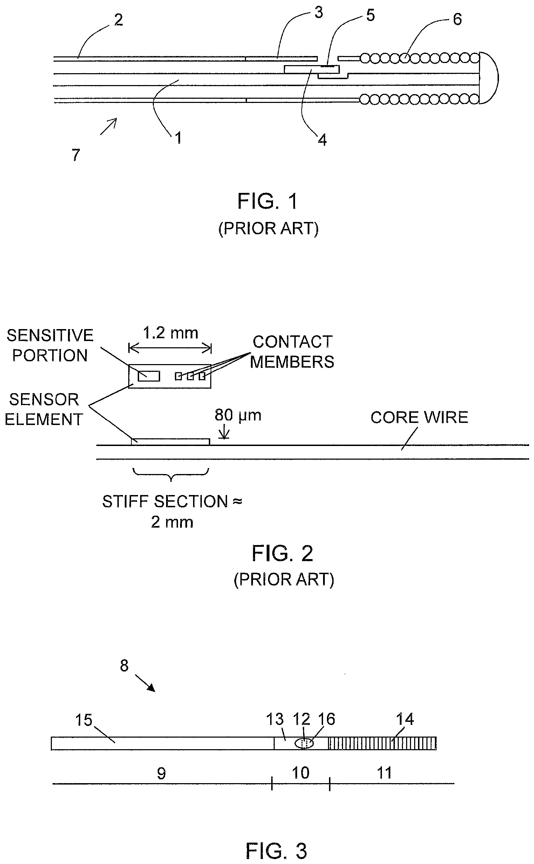

Figure 1 illustrates the distal part of a sensor guide wire 7 according to the prior art, see e.g. the above-mentioned U.S. Patent No. 6,167,763. The sensor guide wire 7 comprises a core wire 1, a hollow tube 2, a hollow jacket 3, a sensor element 4 with a sensitive portion 5, e.g. a pressure sensitive portion, and a coil 6. The core wire 1 is at least partly disposed inside the hollow tube 2 and extends through the jacket 3 and into the coil 6. The sensor element 4, comprising the sensitive portion 5, is mounted on the core wire 1 within the jacket 3, and is connected to an electronic unit (not shown in the figure) via one or several electrical leads, e.g. so-called microcables (not shown in the figure).

Figure 2 is a schematic side view of a core wire and a sensor element according to prior art. In the figure the distal end of the core wire is disclosed provided with a sensor element, e.g. in the form of a rectangular chip which is approximately 1.2 mm long and 80 µm thick. The sensor element includes a sensitive portion and a number of contact elements for connection to microcables. The sensor element is normally enclosed in a jacket which is obviated in the figure for sake of simplicity. As discussed in the background section this known construction results in a rather long stiff section of the sensor guide wire which might cause bending artefacts of the sensor element that could influence the measurement accuracy.

In figure 3 is disclosed a side view of a sensor guide wire 8 for intravascular measurements of physiological variables, or externally generated signals, e.g. electrical and/or magnetic field signals, in a living body according to the present invention, having a proximal region 9, a distal sensor region 10 and a tip region 11. The sensor guide wire 8 comprises a core wire (not shown) having a longitudinal axis parallel to the longitudinal axis of the sensor guide wire, a sensor element 12 arranged in the distal sensor region, which has a sensitive portion (not shown), e.g. a pressure sensitive portion, for measuring the physiological variable, or the external signal, and to generate a sensor signal in response of the variable/external signal, and a jacket 13, accommodating at least a portion of the sensor element 12. At least one signal transmitting microcable (not shown) may be connected to the sensor element 12, and running along the sensor guide wire 8. The sensor guide wire 8 further comprises a coil 14 arranged in the tip region 11 and a hollow tube 15 at least partly enclosing the signal transmitting microcable(s), in the proximal region 9. According to this preferred embodiment, the jacket 13 is provided with a jacket opening 16 in the sensor region 10, at the site where the pressure sensitive portion is arranged.

Figure 4 is a perspective view illustrating the present invention. In the figure only the core wire and the sensor element are shown in order to fully illustrate the invention.

The sensor element has an essentially planar main surface and has its maximal extension in the plane of the main surface, and a thickness perpendicular to the plane of the main surface, wherein the sensor element is arranged in an essentially perpendicular relation to the core wire with regard to the planar main surface of the sensor element.

The sensor element is provided with a through-going opening in the perpendicular direction with regard to the main surface, wherein the sensor element is mounted in relation to the core wire such that said core wire extends through said through-going opening and such that the plane of the sensor element is essentially perpendicular to the longitudinal axis of the core wire.
The angle between the planar main surface and the axis of the core wire is in the range of 80-100 degrees.

According to a first embodiment the through-going opening is a hole, preferably a circular hole which is illustrated in figures 4 and 5.

Figure 5 is a top view of the sensor element according to the first embodiment. In this embodiment the sensor element is provided with a circular hole for receiving the core wire, a sensor portion and a number of contact members including contact elements for establishing electrical connection to one or many micro-cables. In the illustrated sensor element three contact members are included.

According to another embodiment a notch in the periphery of the sensor element is arranged such that the micorcables may pass the sensor element and an electrical connection may be provided at the distal side of the sensor element. This embodiment is illustrated by figure 6. This embodiment simplifies the mounting procedure in that the sensor element may be horizontally arranged when the microcables are connected (by e.g. fusing or soldering) to the contact elements and the sensor element is then turned to a vertical position in relation to the core wire and the insulated microcables pass to the distal side via the notch. The arrangement of a notch in the periphery of the sensor element allowing the microcables to pass the sensor element may be applied to all embodiments described herein, wherever it is applicable. The notch is illustrated in figure 6, and also in figure 10.

The present invention is also applicable in embodiments where the communication between the sensor element and an external unit is wireless. In that case the sensor element is provided with a processing and transmitting unit adapted to process the measured physiological signal and to transmit the processed signal to the external unit. In that case no contact members are required.

According to a second and a third embodiment of the present invention, which are illustrated by figures 8 and 9, the through-going opening is a radial slit having a predetermined width related to the width of the core wire extending from the periphery to the centre of the sensor element. The width is such that the core wire easily may be arranged within the slit. One advantage with these embodiments is that the manufacturing and mounting processes are simplified. Another advantage is that the microcables also may be arranged in the radial slit. In that regard the core wire is first arranged and the microcables are then arranged in the remaining space in the radial slit between the core wire and the sensor element periphery.

The second embodiment is illustrated in figure 8 where the sensor element has a square peripheral shape where the slit extends from one of the sides to the centre of the sensor element.

The third embodiment is illustrated in figure 9 where the sensor element has a circular peripheral shape where the slit extends from the side to the centre of the sensor element.

Figure 10 is a top view illustrating a fourth embodiment of the present invention where the sensor element has a rectangular peripheral shape and is provided with a notch on one of its sides where the microcables are arranged such that they then pass the sensor element and provide for connection to the contact members at the distal side of the sensor element.

Thus, the planar main surface of the sensor element has a circular or elliptical shape, or as an alternative, the planar main surface of said sensor element has a rectangular or square shape. These shapes are applicable for all embodiments described herein. Naturally, within the scope of the present invention as defined by the claims, other shapes are possible, e.g. triangular, or non-symmetrical shapes.

The through-going opening has a size such that said core wire extends through the opening when the sensor element is mounted on the core wire.

Figures 11a-11d are top views of a circular sensor element schematically illustrating different cross-sectional shapes of the core wire and opening.

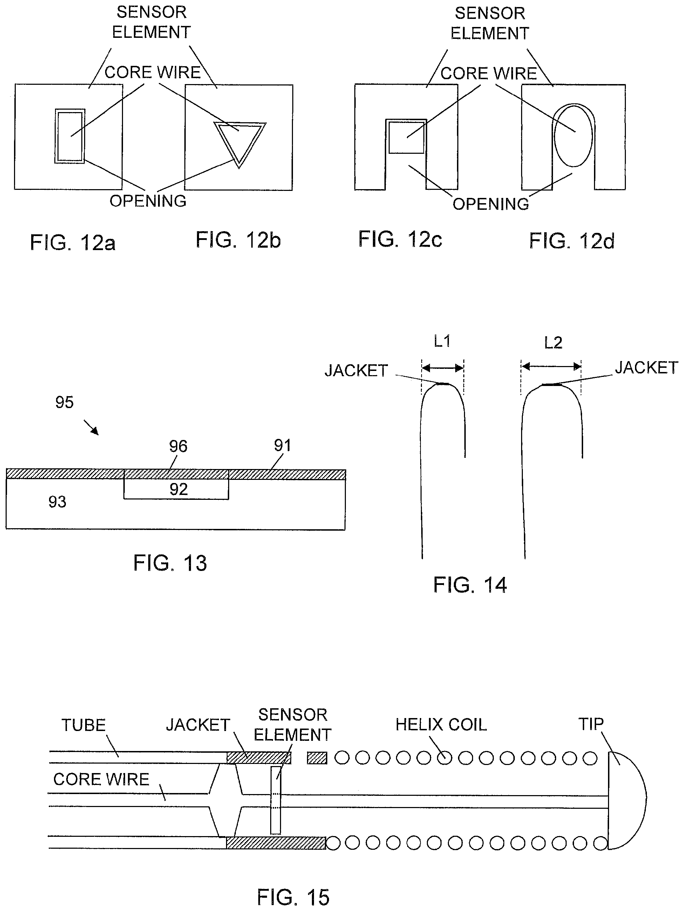

Figures 12a-12d are top views of a rectangular sensor element schematically illustrating different cross-sectional shapes of the core wire and opening.

According to the embodiments illustrated in figures 11a-11d and 12a-12d the cross-sectional shape of the opening is adapted to the cross-sectional shape of core wire where the sensor element is to be arranged. Thereby the sensor element may be safely positioned, e.g. rotation is obviated. Another advantage is that the mounting of the sensor element is simplified in that that the cross-sectional shape of the opening and corewire can be chosen such that there is only one way to mount the sensor element at the core wire.

In the figures the core wire is given a rectangular, a triangular, a square and an elliptical shape, respectively. The cross-sectional shape of the core wire may be achieved e.g. by grinding and is preferably made only where the sensor element should be mounted.

Advantageously, the opening for the core wire, or the radial slit and/or the notch are etched. Other manufacturing methods are naturally possible within the scope of the present invention. Below, a Silicon-On-Insulator (SOI) manufacturing method will briefly be described which method is applicable when manufacturing the sensor element according to the present invention.

Preferably, the sensor element is mounted such that the sensitive portion faces the distal direction of the sensor guide wire. It is also possible to mount the sensor element such that the sensitive portion faces the proximal direction of the sensor guide.

Advantageously, the sensor element is attached to the core wire by glue, or by welding or soldering.

The sensor element is adapted to measure one or many of the physiological variables pressure, temperature, flow. In addition the sensor element may be adapted to measure external signals, such as electromagnetic signals, magnetic signals, radio signals, etc, e.g. to determine the position of the sensor element.

The maximal extension of the sensor element is at least three times larger than the thickness. The outer diameter of the core wire is preferably in the range of 60 - 200 µm, the thickness of the sensor element is less than 100 µm, preferably 80 µm and the maximal extension of the sensor element is in the range of 300 - 400 µm, preferably 360 µm.

Pressure sensors, which is one preferred application of the sensor wire according to the present invention, used in the context of measuring intracoronary pressure often contain a deflectable diaphragm. The two main types of such pressure sensors are absolute pressure sensors and differential or relative pressure sensors. In an absolute pressure sensor the diaphragm is usually mounted across a small cavity wherein a reference pressure, usually vacuum pressure, exists, and the pressure to be measured acts on the opposing surface of the diaphragm. A differential pressure sensor measures the difference of two pressures acting on opposing sides of the diaphragm.
The movement or deformation of the diaphragm can be sensed in different ways, such as by measuring the changes of electric characteristics of a piezoresistive body, the changes of resistance of an electrical conductor or the change of capacitance of a suitable adapted capacitor coupled to the movement of the diaphragm and thereby being in varied forced or strained states.

Absolute pressure sensors need a hermetic sealing of a relatively small cavity at the active diaphragm to get a reference pressure, preferably a vacuum enclosure. This can be accomplished on a wafer using e.g. silicon wafer bonding under vacuum conditions.

Generally, for example for use in a sensor guide wire as described above, a small piezoresistive absolute pressure sensor is desired, having a high pressure sensitivity, a controlled temperature behaviour and a high long term stability. It should not be affected by environmental changes, such as humidity or possible temperature fluctuations. Also, a manufacturing process suitable for high volume production and with a high yield is preferred.

Recently, micromachining techniques have been developed and refined for producing integrated miniaturized pressure sensors of semiconductor material, providing several advantages over traditional pressure sensors: low cost, high degree of performance and reliability, better signal/noise ratio, and greater reproducibility.

Several pressure sensors based on silicon-on-insulator (SOI) substrates have been proposed. For example, U.S. Patent Nos. 6,131,466, 5,510,276, 5,095,401, and 7,207,227, disclose such sensors. In U.S. Patent No. 7,207,227, a method of manufacturing a pressure sensor is described, wherein a cavity is formed in an SOI substrate, and thereafter a second silicon wafer is bonded to the first to seal the cavity. After several etching and deposition steps, a sensor complete with electrical strain gauge is produced.

Figure 13 is a schematic cross-sectional view illustrating a conventional pressure sensor chip 95 (that corresponds to the sensor element referred to in the present application) based on an SOI substrate. The pressure sensor chip 95 includes a crystalline silicon substrate 93, a cavity recess 92 formed in the crystalline silicon substrate 93, and a crystalline silicon layer 91 forming a diaphragm 96 over the cavity recess 92. A certain pressure exerted on the diaphragm 96 from the surrounding medium will thereby correspond to a certain stretching of the diaphragm 96 and thereby to a certain resistance of piezoresistive elements (not shown in figure 10), disposed on the diaphragm.

Figure 14 illustrates the improved flexibility of the distal part of the sensor guide wire according to the present invention. In the figure the distal portion of the sensor wire is schematically shown; the left illustration shows the distal part according to the present invention where the short stiff portion of the jacket, approximately 1.1 mm, improves the flexibility in comparison to the presently used sensor guide wire, which is illustrated by the right illustration, where the stiff portion is considerably longer. In the figure the maximal bending of the sensor guide wires are shown and the lengths L1 and L2 (which are related to the bending radius) are indicated, which very clearly illustrate the improved flexibility and ability to easily insert the guide wire into tortuous vessels.

And finally, figure 15 shows a side view of the distal portion of a sensor guide wire according to the present invention. The core wire is provided with a short portion where the diameter is increased where the jacket and a proximal tube is attached to the core wire. In the figure is also shown the sensor element being protected by the jacket, having a typical longitudinal extension of 1-1.5 mm, preferably 1.1 mm. The jacket is provided with an opening through which the sensor element is brought into contact with e.g. the blood. The most distal part of the sensor guide wire is preferably provided with a flexible helix wounded coil and a protective tip.

The present invention is not limited to the above-described preferred embodiments. Various alternatives, modifications and equivalents may be used. Therefore, the above embodiments should not be taken as limiting the scope of the invention, which is defined by the appending claims.

## Claims

1. Sensor guide wire for intravascular measurements of physiological variables in a living body or of external signals, which sensor guide wire (8) has a proximal i region (9), distal sensor region; (10) and a tip region (11), the sensor guide wire comprises a core wire having a longitudinal axis parallel to the longitudinal axis of the sensor guide wire, and a sensor element (12) arranged in the distal sensor region, the sensor element has a sensitive portion facing the distal or proximal direction of the sensor guide wire for measuring the physiological variable/external signal and to generate a sensor signal in response to said variable/external signal, and the sensor element is provided with a through-going opening in the perpendicular direction with regard to the main surface, wherein said sensor element is mounted in relation to the core wire such that said core wire extends through said through-going opening and such that the plane of the sensor element is essentially perpendicular to the longitudinal axis of the core wire; wherein said sensor element has an essentially planar main surface and has its maximal extension in the plane of the main surface, and a thickness perpendicular to the plane of the main surface, wherein the sensor element is arranged in an essentially perpendicular relation to said core wire with regard to the planar main surface of the sensor element.

2. Sensor guide wire according to claim 1, wherein the angle between the planar main surface and the axis of the core wire is in the range of 80-100 degrees.

3. Sensor guide wire according to claims 1 or 2, wherein said through-going opening is a hole.

4. Sensor guide wire according to claims 1 or 2, wherein said through-going opening is a radial slit having a predetermined width related to the width of the core wire extending from the periphery to the centre of the sensor element.

5. Sensor guide wire according to any preceding claim, wherein the maximal extension of the sensor element is at least three times larger than the thickness.

6. Sensor guide wire according to any of claims 2-5, wherein said through-going opening has a size such that said core wire extends through said opening when the sensor element is mounted on the core wire.

7. Sensor guide wire according to any of claims 2-6, wherein core wire has a cross-sectional shape that is adapted to the cross-sectional shape of the opening of the sensor element, such that rotation of the sensor element in relation to the core wire is obviated.

8. Sensor guide wire according to any preceding claim, wherein an outer diameter of the core wire is in the range of 60 - 200 µm, the thickness of the sensor element is less than 100 µm, preferably 80 µm and the maximal extension of the sensor element is in the range of 300 - 400 µm, preferably 360 µm.

9. Sensor guide wire according to any preceding claim, wherein said guide wire further comprises a jacket (13) accommodating at least a portion of said sensor element, said jacket is provided with an opening enabling said sensitive portion to measure the physiological variable, and that said jacket has a longitudinal extension of 1-1.5 mm.

10. Sensor guide wire according to any preceding claim, wherein said sensor element is mounted such that the sensitive portion faces the distal direction of the sensor guide wire.

11. Sensor guide wire according to any preceding claim, wherein the sensor element is provided with one or several contact members for establishing electrical connection to one or several micro-cables.

12. Sensor guide wire according to any preceding claim, wherein the planar main surface of said sensor element has a circular or elliptical shape.

13. Sensor guide wire according to any of claims 1-11, wherein the planar main surface of said sensor element has a rectangular or square shape.

14. Sensor guide wire according to any preceding claim, wherein the periphery of the sensor element is provided with a notch, such that microcables may pass the sensor element.

15. Sensor guide wire according to any preceding claim, wherein said sensor element is attached to said core wire by glue.

16. Sensor guide wire according to any preceding claim, wherein said sensor element is a pressure sensor.

## Patentansprüche

1. Sensorführungsdraht zur intravaskulären Messung physiologischer Variablen in einem lebenden Körper oder von externen Signale, wobei der Sensorführungsdraht (8) einen proximalen Bereich (9) einen distalen Sensorbereich (10) und einen Spitzenbereich (11) aufweist, wobei der Sensorführungsdraht einen Kerndraht mit einer zur Längsachse des Sensorführungsdraht parallelen Längsachse und einem im distalen Sensorbereich angeordneten Sensorelement (12) umfasst, wobei das Sensorelement einen empfindlichen Abschnitt aufweist, der der distalen oder proximalen Richtung des Sensorführungsdrahtes zugewandt ist, um das physiologische variable/externe Signal zu messen und ein Sensorsignal als Reaktion auf das variable/externe Signal zu erzeugen, und das Sensorelement mit einer Durchgangsöffnung in senkrechter Richtung in Bezug auf die Hauptfläche versehen ist, wobei das Sensorelement in Bezug auf den Kerndraht so angebracht ist, dass sich der Kerndraht durch die Durchgangsöffnung erstreckt, und dass die Ebene des Sensorelements im Wesentlichen senkrecht zur Längsachse des Kerndrahtes ist;
wobei das Sensorelement eine im wesentlichen ebene Hauptfläche aufweist, und seine maximale Ausdehnung in der Ebene der Hauptfläche und eine Dicke senkrecht zur Ebene der Hauptfläche aufweist, wobei das Sensorelement in einer im wesentlichen senkrechten Beziehung zu dem Kerndraht in Bezug auf die ebene Hauptfläche des Sensorelements angeordnet ist.

2. Sensorführungsdraht nach Anspruch 1, wobei der Winkel zwischen der ebenen Hauptfläche und der Achse des Kerndrahtes im Bereich von 80 bis 100 Grad liegt.

3. Sensorführungsdraht nach Anspruch 1 oder 2, wobei die Durchgangsöffnung ein Loch ist.

4. Sensorführungsdraht nach Anspruch 1 oder 2, wobei die Durchgangsöffnung ein radialer Schlitz mit einer vorbestimmten Breite ist, die sich auf die Breite des Kerndrahtes bezieht, der sich vom Umfang zur Mitte des Sensorelements erstreckt.

5. Sensorführungsdraht nach einem der vorhergehenden Ansprüche, wobei die maximale Ausdehnung des Sensorelements mindestens dreimal größer als die Dicke ist.

6. Sensorführungsdraht nach einem der Ansprüche 2 bis 5, wobei die Durchgangsöffnung eine solche Größe aufweist, dass sich der Kerndraht durch die Öffnung erstreckt, wenn das Sensorelement auf dem Kerndraht aufgebracht ist.

7. Sensorführungsdraht nach einem der Ansprüche 2 bis 6, wobei der Kerndraht eine Querschnittsform aufweist, die an die Querschnittsform der Öffnung des Sensorelements angepasst ist, so dass eine Drehung des Sensorelements in Bezug auf den Kerndraht vermieden wird.

8. Sensorführungsdraht nach einem der vorhergehenden Ansprüche, wobei ein Außendurchmesser des Kerndrahtes im Bereich von 60 bis 200 µm liegt, die Dicke des Sensorelements weniger als 100 µm, vorzugsweise 80 µm beträgt, und die maximale Ausdehnung des Sensorelements im Bereich von 300 - 400 µm, vorzugsweise 360 µm liegt.

9. Sensorführungsdraht nach einem der vorhergehenden Ansprüche, wobei der Führungsdraht ferner einen Mantel (13) umfasst, der mindestens einen Teil des Sensorelements aufnimmt, wobei der Mantel mit einer Öffnung versehen ist, die es dem empfindlichen Abschnitt ermöglicht, die physiologische Variable und den Mantel zu messen, und dass der Mantel eine Längsausdehnung von 1-1,5 mm hat.

10. Sensorführungsdraht nach einem der vorhergehenden Ansprüche, wobei das Sensorelement so angebracht ist, dass der empfindliche Abschnitt der distalen Richtung des Sensorführungsdrahtes zugewandt ist.

11. Sensorführungsdraht nach einem der vorhergehenden Ansprüche, wobei das Sensorelement mit einem oder mehreren Kontaktelementen zum Herstellen einer elektrischen Verbindung zu einem oder mehreren Mikrokabeln versehen ist.

12. Sensorführungsdraht nach einem der vorhergehenden Ansprüche, wobei die ebene Hauptfläche des Sensorelements eine kreisförmige oder elliptische Form aufweist.

13. Sensorführungsdraht nach einem der Ansprüche 1 bis 11, wobei die ebene Hauptfläche des Sensorelements eine rechteckige oder quadratische Form aufweist.

14. Sensorführungsdraht nach einem der vorhergehenden Ansprüche, wobei der Umfang des Sensorelements mit einer Kerbe versehen ist, so dass Mikrokabel das Sensorelement passieren können.

15. Sensorführungsdraht nach einem der vorhergehenden Ansprüche, wobei das Sensorelement durch Kleben an dem Kerndraht befestigt ist.

16. Sensorführungsdraht nach einem der vorhergehenden Ansprüche, wobei das Sensorelement ein Drucksensor ist.

## Revendications

1. Fil-guide pour capteur pour des mesures intravasculaires de variables physiologiques dans un corps vivant ou de signaux externes, lequel fil-guide pour capteur (8) a une région proximale (9), une région capteur distale (10) et une région d'extrémité (11), le fil-guide pour capteur comprend un fil de noyau ayant un axe longitudinal parallèle à l'axe longitudinal du fil-guide pour capteur, et un élément capteur (12) agencé dans la région capteur distale, l'élément capteur a une partie sensible faisant face à la direction distale ou proximale du fil-guide pour capteur pour mesurer une/un variable physiologique/signal externe et pour générer un signal de capteur en réponse auxdits variable/signal externe, et l'élément capteur est pourvu d'une ouverture traversante dans la direction perpendiculaire par rapport à la surface principale, où ledit élément capteur est monté par rapport au fil de noyau de telle sorte que ledit fil de noyau s'étend à travers ladite ouverture traversante et de telle sorte que le plan de l'élément capteur soit essentiellement perpendiculaire à l'axe longitudinal du fil de noyau;
où ledit élément capteur a une surface principale essentiellement plane et a une extension maximale dans le plan de la surface principale, et une épaisseur perpendiculaire au plan de la surface principale, où l'élément capteur est agencé dans une relation essentiellement perpendiculaire audit fil de noyau par rapport à la surface principale plane de l'élément capteur.

2. Fil-guide pour capteur selon la revendication 1, dans lequel l'angle entre la surface principale plane et l'axe du fil de noyau est dans la plage de 80 à 100 degrés.

3. Fil-guide pour capteur selon la revendication 1 ou 2, dans lequel ladite ouverture traversante est un trou.

4. Fil-guide pour capteur selon la revendication 1 ou 2, dans lequel ladite ouverture traversante est une fente radiale ayant une largeur prédéterminée liée à la largeur du fil de noyau s'étendant de la périphérie au centre de l'élément capteur.

5. Fil-guide pour capteur selon l'une quelconque des revendications précédentes, dans lequel l'extension maximale de l'élément capteur est au moins trois fois plus grande que l'épaisseur.

6. Fil-guide pour capteur selon l'une quelconque des revendications 2 à 5, dans lequel ladite ouverture traversante a une taille telle que ledit fil de noyau s'étend à travers ladite ouverture lorsque l'élément capteur est monté sur le fil de noyau.

7. Fil-guide pour capteur selon l'une quelconque des revendications 2 à 6, dans lequel le fil de noyau a une forme de section transversale qui est adaptée à la forme de section transversale de l'ouverture de l'élément capteur, de telle sorte que la rotation de l'élément capteur par rapport à le fil de noyau est évitée.

8. Fil-guide pour capteur selon l'une quelconque des revendications précédentes, dans lequel un diamètre extérieur du fil de noyau est compris entre 60 et 200 µm, l'épaisseur de l'élément capteur est inférieure à 100 µm, de préférence 80 µm et l'extension maximale de l'élément capteur est dans la plage de 300 - 400 µm, de préférence 360 µm.

9. Fil-guide pour capteur selon l'une quelconque des revendications précédentes, dans lequel ledit fil-guide comprend en outre une gaine (13) recevant au moins une partie dudit élément capteur, ladite gaine est pourvue d'une ouverture permettant à ladite partie sensible de mesurer la variable physiologique, et ladite gaine a une extension longitudinale de 1-1,5 mm.

10. Fil-guide pour capteur selon l'une quelconque des revendications précédentes, dans lequel ledit élément capteur est monté de telle sorte que la partie sensible fait face à la direction distale du fil-guide pour capteur.

11. Fil-guide pour capteur selon l'une quelconque des revendications précédentes, dans lequel l'élément capteur est pourvu d'un ou plusieurs organes de contact pour établir la connexion à un ou plusieurs micro-câbles.

12. Fil-guide pour capteur selon l'une quelconque des revendications précédentes, dans lequel la surface principale plane dudit élément capteur a une forme circulaire ou elliptique.

13. Fil-guide pour capteur selon l'une quelconque des revendications 1 à 11, dans lequel la surface principale plane dudit élément capteur a une forme rectangulaire ou carrée.

14. Fil-guide pour capteur selon l'une quelconque des revendications précédentes, dans lequel la périphérie de l'élément capteur est pourvu d'une encoche, de sorte que des micro-câbles peuvent passer l'élément capteur.

15. Fil-guide pour capteur selon l'une quelconque des revendications précédentes, dans lequel ledit élément capteur est attaché audit fil de noyau par de la colle.

16. Fil-guide pour capteur selon l'une quelconque des revendications précédentes, dans lequel ledit élément capteur est un capteur de pression.
